# FASCICULE DE BREVET EUROPEEN

(11) **EP 1 496 765 B1**
(45) Date de publication et mention de la délivrance du brevet: **26.10.2005**
(21) Numéro de dépôt: 03740613.9
(22) Date de dépôt: 15.04.2003
(51) Int. Cl.: A43B 7/22

(54) **SEMELLE ORTHOPEDIQUE PROPRIOCEPTIVE COMPRENANT DES MOYENS MODULAIRES DE CORRECTION**
PROPRIOZEPTIVE ORTHOPÄDISCHE EINLAGE MIT MODULAREM KORREKTURMITTEL
PROPRIOCEPTIVE ORTHOPEDIC SOLE COMPRISING MODULAR CORRECTION MEANS

(30) Priorité: 15.04.2002 FR 0204674
(43) Date de publication de la demande: 19.01.2005
(73) Titulaire: Chenut, Pascal, 21220 Semezanges (FR)
(72) Inventeur: CHENUT, Pascal, F-21220 Semezanges (FR); DEREN, Bernard, F-21121 Ahuy (FR); DOUHAIRE, Jean, F-21000 Dijon (FR)
(74) Mandataire: Honoré, Anne-Claire
(86) Numéro de dépôt international: PCT/FR2003/001201
(87) Numéro de publication internationale: WO 2003/086127

(56) Documents cités:
- CA-A- 2 075 265
- DE-U- 1 668 262
- FR-A- 2 676 918
- GB-A- 181 445
- US-A- 6 105 283

## Description

La présente invention concerne une semelle orthopédique proprioceptive pour le rétablissement et/ou la préservation d'une dynamique correcte de la marche ou de la course comprenant des moyens modulaires de correction d'un varus, d'un valgus, d'une abduction ou d'une adduction du pied.

Dans le domaine des semelles destinées à être insérées dans des chaussures, on connaît bien des semelles dites proprioceptives destinées au rétablissement et/ou à la préservation d'une dynamique correcte de la marche ; c'est le cas, par exemple, du brevet français FR 2676918 du même Déposant. La semelle comporte des moyens pour solliciter, dès l'attaque du pas normalement effectué par le talon, les récepteurs myo-articulaires situés entre l'astragale et le calcanéum, le tonus musculaire ainsi mis en jeu par cette impulsion initiale étant ensuite canalisé par des moyens guidant le déroulement du pied sur l'axe physiologique de la marche. A cet effet, la semelle comporte notamment sur sa face supérieure une console de section droite à sa partie supérieure, et d'épaisseur croissant depuis le talon jusqu'à une zone située sensiblement à l'aplomb vertical du col de l'astragale, des moyens compensateurs et incitateurs latéraux sous-cuboïdien et sous-scaphoïdien agissant en rappel des torsions en varus ou en valgus du pied, et un moyen axial médiotarsien destiné à répartir l'appui sous les palettes métatarsiennes.

Ces semelles qui sont exclusivement destinées à rendre la marche plus confortable sont inopérantes en cas de défaut d'appui trop important dans les trois plans de l'espace, dépassant l'action proprioceptive de la semelle, lors de la pratique d'un sport tel que la course à pied par exemple. Ce défaut d'appui procure un varus ou supination, un valgus ou pronation, une abduction ou une adduction du pied entraînant un déséquilibre articulaire, tendineux et musculaire qui est à l'origine de nombreuses douleurs ressenties au cours d'une pratique sportive.

A cet égard, on connaît déjà des semelles orthopédiques particulièrement destinées à la pratique sportive et comprenant des moyens de correction d'un varus, d'un valgus, d'une abduction ou d'une adduction du pied ; c'est le cas, par exemple, du brevet américain US 4.841.648 décrivant une semelle orthopédique modulaire. La semelle qui est obtenue dans une matière résiliente comporte sur sa face supérieure plusieurs zones de correction comprenant des boucles d'un dispositif de fixation du type "velcro" (marque déposée) sur lesquels sont solidarisés des éléments de correction obtenus dans un matériau également résilient dont la face inférieure est munie de crochets aptes à coopérer avec les boucles du velcro et dont la forme correspond à la forme des zones de correction. Les différents éléments de correction sont avantageusement référencés par un code couleur, un code numérique ou une étiquette dont les propriétés sont rappelées dans une notice jointe à la semelle permettant à l'utilisateur de positionner un ou plusieurs éléments de correction sur la semelle en fonction des douleurs qu'il ressent lors de sa pratique sportive.

Les éléments de correction de ce type de semelle orthopédique présentent l'inconvénient de glisser sur la face supérieure de la semelle lorsque l'utilisateur effectue une course à pied par exemple de sorte que lesdits éléments sont rapidement inefficaces. En effet, lors d'une pratique sportive, la transpiration des pieds combinés à la chaleur corporelle et aux contraintes de cisaillement appliquées par le pied sur les éléments de correction engendrant leur déplacement, ce qui les rend inopérants, voire dangereux en provoquant des défauts d'appui du pied qui sont susceptibles d'entraîner des traumatismes musculaires, tendineux ou osseux.

Des éléments de correction amovibles sont connus de DE 1 668 262 U.

L'un des buts de l'invention est de remédier à tous ces inconvénients en proposant une semelle orthopédique proprioceptive pour le rétablissement d'une dynamique correcte de la marche ou de la course comprenant des moyens modulaires de correction d'un varus, d'un valgus, d'une abduction ou d'une adduction du pied.

A cet effet, et conformément à l'invention, il est proposé une semelle orthopédique proprioceptive modulaire pour le rétablissement et/ou la préservation d'une dynamique correcte de la marche ou de la course ; cette semelle, obtenue dans un matériau élastique est remarquable en ce qu'elle comporte d'une part, sur sa face supérieure, des moyens pour solliciter, dès l'attaque du pas habituellement effectué par le talon, les récepteurs articulaires situés entre l'astragale et le calcanéum et des moyens guidant le déroulement du pied sur l'axe physiologique de la marche et d'autre part, sur sa face inférieure, au moins un élément de correction amovible apte à procurer une abduction ou une adduction lors d'une ouverture de pas déficitaire ou respectivement trop importante et/ou à corriger un varus ou supination et/ou un valgus ou une pronation.

On a constaté, de manière surprenante, que la combinaison des éléments de correction positionnés sur la face inférieure et des moyens positionnés sur la face supérieure de la semelle sollicitant les récepteurs articulaires situés entre l'astragale et le calcanéum et guidant le déroulement du pied sur l'axe physiologique de la marche, procure une meilleure correction d'un valgus et/ou d'un varus et/ou d'une abduction et/ou d'une adduction du pied tout en utilisant des éléments de correction de faibles épaisseurs réduisant ainsi l'impression d'inconfort des semelles orthopédiques de l'art antérieur.

De manière particulièrement avantageuse, la semelle comprend sur sa face inférieure au moins un évidement positionné le long du bord interne et/ou externe de ladite semelle, dans lequel l'élément de correction amovible obtenu dans un matériau plus rigide que le matériau de la semelle, et de forme correspondant à la forme de l'évidement, est apte à être positionné de telle sorte que les éléments de correction ne puissent pas se déplacer sous la semelle en courant ou en marchant. De plus, on observera que les éléments de correction étant positionnés sur la face inférieure de la semelle, ils ne sont pas au contact du pied évitant ainsi toute lésion cutanée plantaire telle que des coupures, des ampoules, des crevasses, ou similaires contrairement aux semelles de l'art antérieur.

D'autres avantages et caractéristiques ressortiront mieux de la description qui va suivre de plusieurs variantes d'exécution, données à titre d'exemples non limitatifs de la semelle orthopédique proprioceptive conforme à l'invention, en référence aux dessins annexés sur lesquels :
- la figure 1 est une vue de dessus de la semelle orthopédique suivant l'invention,
- la figure 2 est une vue de côté de la semelle orthopédique suivant l'invention,
- la figure 3 est une vue de dessous de la semelle orthopédique suivant l'invention,
- la figure 4 est une vue en coupe suivant l'axe IV-IV de la semelle orthopédique représentée sur la figure 3,
- les figures 5a à 8a sont des vues de dessus des éléments de correction amovibles de la semelle orthopédique suivant l'invention,
- les figures 5b à 8b sont des vues de côté des éléments de correction amovibles de la semelle orthopédique représentés sur les figures 5a à 8a,
- la figure 9 est une vue de dessous d'une variante d'exécution de la semelle orthopédique conforme à l'invention, dans laquelle les éléments de correction ne sont pas représentés.

Pour de simples raisons de simplification de la description, on ne décrira qu'une semelle, conforme à l'invention, correspondant au pied gauche par exemple, la semelle droite s'en déduisant par symétrie.

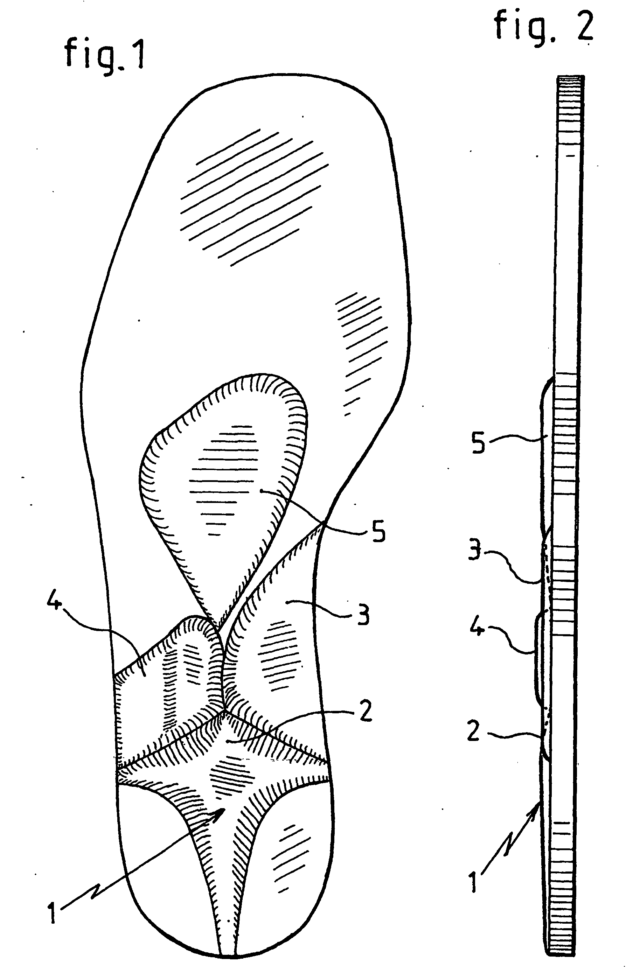

En référence aux figures 1 et 2, la semelle comporte sur sa face supérieure, des moyens pour solliciter, dès l'attaque du pas habituellement effectué par le talon, les récepteurs articulaires situés entre l'astragale et le calcanéum et des moyens guidant le déroulement du pied sur l'axe physiologique de la marche. Ces moyens consistent essentiellement dans un canal profilé, appelé console 1, et un jeu d'éléments profilés 3, 4, 5 répartis tout le long de la semelle pour matérialiser un rail autour duquel le pied est guidé.

La console 1 s'étend longitudinalement depuis le talon jusqu'à l'extrémité antérieure du calcanéum, juste à l'aplomb vertical du col de l'astragale. Ladite console 1 présente une épaisseur croissante depuis le talon jusqu'à son extrémité antérieure 2. A titre d'exemple particulier, la hauteur de la console 1 passe progressivement de 1 mm à 2 mm depuis le talon jusqu'à son extrémité antérieure 2.

Le jeu d'éléments profilés 3, 4, 5 est constitué d'arrière en avant, c'est-à-dire depuis le talon vers la pointe du pied, d'un élément profilé sous-scaphoïdien 3, d'un élément profilé sous-cuboïdien 4 et d'un moyen axial médiotarsien 5. L'élément profilé sous-scaphoïdien 3 prolonge la console 1 vers l'intérieur du pied à la manière d'une hémicoupole. Cet élément profilé sous-scaphoïdien 3 présente dans cet exemple une hauteur d'environ 2 mm et prolonge ainsi l'extrémité antérieure 2 de la console 1. L'élément profilé sous-cuboïdien 4 se présente en vue de dessus, conformément à la figure 1, sous la forme d'une graine de haricot correspondant globalement à la projection de la forme du cuboïde sur la semelle. Cet élément 4 est situé du côté externe de l'élément sous-scaphoïdien 3, sa convexité tournée vers l'arrière, à environ 45° de l'axe longitudinal médian de la semelle. L'épaisseur dudit élément 4 va, en croissant, du côté vers le centre et d'arrière en avant pour atteindre progressivement une hauteur égale à environ 4 mm. Le moyen médiotarsien 5 présente une forme obovale, c'est-à-dire grossièrement en forme de goutte d'eau, s'élargissant vers l'avant et se terminant juste devant les têtes métatarsiennes du pied. Cet élément médiotarsien 5 est bombé ; sa hauteur varie longitudinalement depuis une hauteur de 2,5 mm pour atteindre une hauteur maximum de l'ordre de 3,5 mm, à environ deux tiers de sa longueur.

On observera que, lors du déroulement du pas, la console 1 sollicite le calcanéum du pied, qu'il s'agisse d'un pied plat ou d'un pied creux, pour préparer dans de bonnes conditions la suite du pas ; puis les éléments profilés sous-scaphoïdien 3 et/ou sous-cuboïdien 4 qui agissent en stabilisateurs latéraux du pied, incitent le pied à rester dans le rail physiologique de la marche et l'élément médiotarsien 5 prépare la phase digitigrade terminale du pas en répartissant l'appui du pied sous les palettes métatarsiennes de manière à ce que cet appui reste canalisé sur l'axe du deuxième métatarsien par lequel passe l'axe physiologique de la marche.

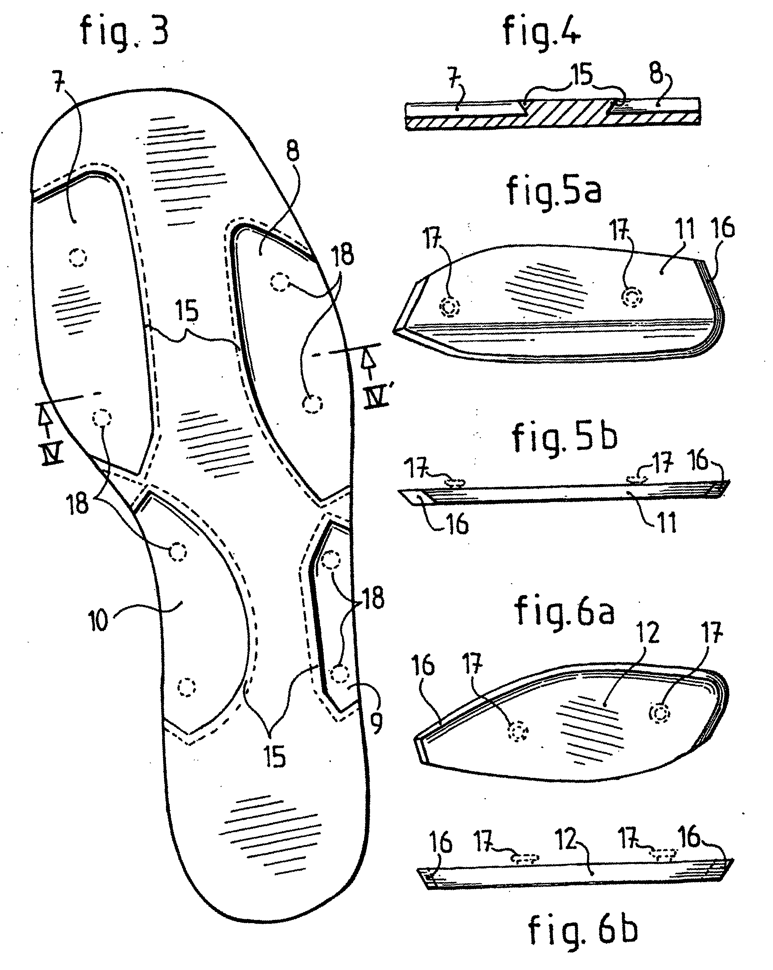

Par ailleurs, la semelle comprend, en référence aux figures 2 et 3, sur sa face inférieure, des évidements 7, 8, 9 et 10 positionnés le long du bord interne et externe de ladite semelle et dans lequel des éléments de correction amovibles 11, 12, 13 et 14 de forme correspondant à la forme de l'évidement sont aptes à être positionnés. La semelle comporte un premier évidement 7 dit anti-abduction de forme sensiblement rectangulaire situé le long du bord interne de la semelle et s'étendant depuis l'arc de la voûte plantaire jusqu'au gros orteil du pied. La section de l'évidement anti-abduction 7, en référence à la figure 3, croît depuis le bord interne de la semelle en direction de la partie médiane de cette dernière sur une courte distance puis décroît. Par ailleurs, en référence à la figure 4, la paroi de l'évidement anti-abduction 7 est incliné vers l'intérieur dudit évidement 7 depuis son fond jusqu'au bord dudit évidement, c'est-à-dire jusqu'à la face inférieure de la semelle, afin de former une lèvre 15 au bord de l'évidement 7. Cet évidement anti-abduction 7 est susceptible d'accueillir un élément de correction de l'abduction 11, représenté sur les figures 5a et 5b, de forme correspondant à la forme de l'évidement anti-abduction 7, c'est-à-dire de forme globalement rectangulaire. Cet élément de correction de l'abdduction 11 comprend à sa périphérie un chanfrein 16 de sorte que la lèvre 15 à la périphérie de l'évidement 7, maintienne en place l'élément amovible dans ledit évidement 7.

Il est bien évident que le bord de l'élément de correction de l'abduction 11 qui est contigu au bord interne de la semelle lorsque ledit élément 11 est introduit dans l'évidement anti-abduction 7 ne comprend pas de chanfrein 16.

Par ailleurs, l'élément de correction de l'abduction 11 est obtenu dans un matériau plus rigide et plus dense que le matériau de la semelle et il est introduit dans l'évidement 7 par déformation élastique de la lèvre 15 de ce dernier. On notera que la forme particulière de l'évidement 7 et de l'élément de correction correspondant 11 empêche tout déplacement intempestif, mais également tout retrait, dudit élément de correction lors de la marche ou de la course. De plus, on observera que l'élément de correction n'est pas au contact du pied évitant ainsi toute lésion cutanée plantaire telle que des coupures, des ampoules, des crevasses, ou similaires.

La semelle, en référence aux figures 2 et 3, comporte, par ailleurs, un second évidement dit anti-adduction 8 de forme globalement rectangulaire, situé sur la face inférieure de la semelle, le long de son bord externe, et s'étendant depuis la voûte plantaire jusqu'à la zone pulpaire du petit doigt de pied, un troisième évidement dit anti-varus ou anti-supination 9 également de forme globalement rectangulaire, situé le long du bord externe de la semelle et s'étendant depuis le cuboïde jusqu'au second évidement anti-adduction 8 et un dernier évidement dit anti-valgus ou anti-pronation 10 en forme d'hémicoupole situé le long du bord interne de la semelle sous la voûte plantaire. De la même manière que précédemment, la paroi des évidements anti-adduction 8, anti-varus 9 et anti-valgus 10 est inclinée vers l'intérieur desdits évidements 8, 9 et 10 depuis leur fond jusqu'à leur bord respectif afin de former une lèvre 15 en bordure desdits évidements 8, 9 et 10. Chacun des évidements d'adduction 8, anti-varus 9 et anti-valgus 10 est prévu pour à accueillir un élément de correction respectivement de l'adduction 12, anti-varus 13 et anti-valgus 14, successivement représentés sur les figures 6a,6b, 7a,7b et 8a,8b ; les éléments de correction d'adduction 12 et anti-varus 13 ont des formes globalement rectangulaires et l'élément de correction anti-valgus 14 à la forme d'une hémicoupole. Chacun de ces éléments de correction est plat et est obtenu dans un matériau plus rigide et plus dense que le matériau de la semelle ; ces éléments comprennent en outre en leur périphérie un chanfrein 16 afin d'assurer leur blocage dans leur évidement respectif.

Selon une variante d'exécution de la semelle conforme à l'invention, chacun des éléments de correction 11, 12, 13 et 14 comprend sur sa face supérieure, c'est-à-dire sa face prenant appui sur le fond de son évidement, deux ergots 17 représentés en traits pointillés sur les figures 5a à 8a et 5b à 8b, susceptibles de coopérer avec deux trous correspondants 18 positionnés au fond des évidements 7, 8, 9 et 10 ; les éléments de correction 11, 12, 13 et 14 ne comprennent pas de chanfrein 16 et les évidements 7, 8, 9 et 10 ne comprennent pas de lèvre 15.

Il va de soi que la face supérieure des éléments de correction peut comprendre tout type d'organe mâle ou femelle de fixation susceptible de coopérer avec un organe femelle ou respectivement mâle positionné au fond de l'évidement afin de procurer un attachement de l'élément amovible dans l'évidement sans pour autant sortir du cadre de l'invention.

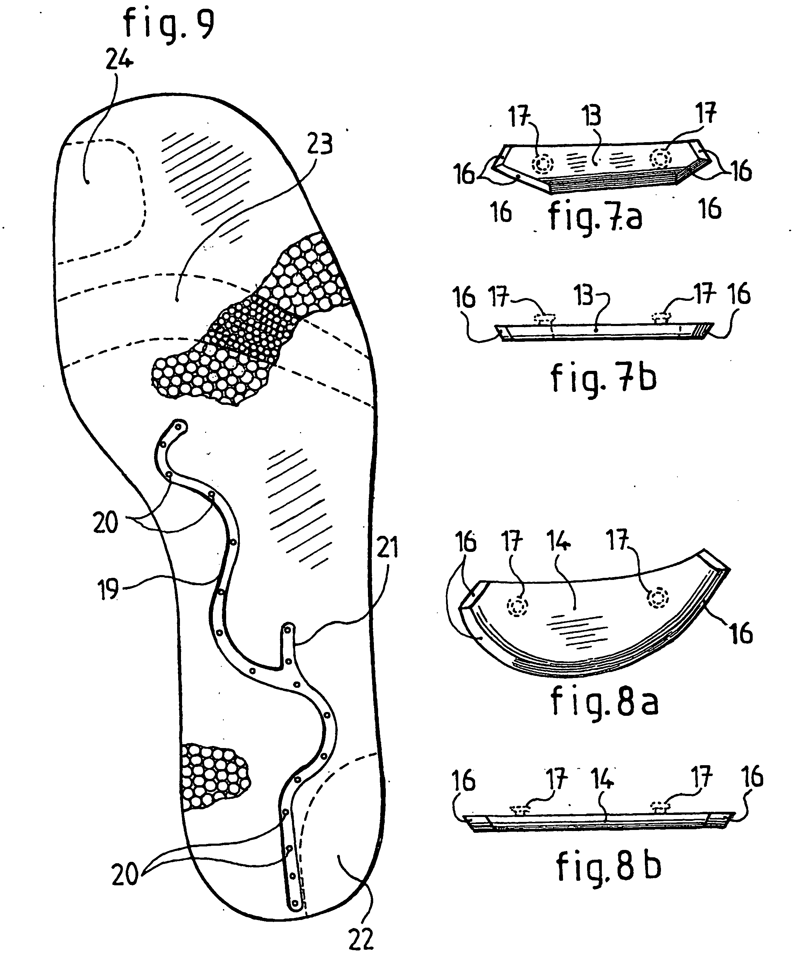

Accessoirement, en référence à la figure 9, la semelle comporte, sur sa face inférieure, un sillon 19 en forme de serpentin qui s'étend sensiblement depuis l'avant pied jusqu'au talon et qui comprend des trous 20 régulièrement espacé débouchant sur la face supérieure de la semelle. Le sillon 19 comprend une petite branche secondaire 21 également munie de trous 20 et qui s'étend sensiblement parallèlement à l'axe longitudinal médian de la semelle sous la voûte plantaire. On notera que ce sillon 19 permet d'évacuer la transpiration vers le fond de la chaussure lors d'une pratique sportive intense par exemple.

Par ailleurs, le talon et l'avant pied de la semelle présente avantageusement une structure alvéolaire, de préférence, en nid d'abeille afin de procurer un plus grand confort. L'intensité de l'appui du pied étant plus importante dans la zone talonnière postero-externe, dans la zone des têtes métatarsienne et dans la zone pulpaire sous le gros orteil au cours du déroulement du pas, la semelle présente dans une zone talonnière postero-externe 22 et/ou dans une zone des métatarses 23 et/ou dans une zone pulpaire sous gros orteil 24, des alvéoles de plus petite taille que les alvéoles du reste de la semelle.

On notera que les éléments profilés positionnés sur la face supérieure de la semelle présentent une hauteur n'excédant pas quelques millimètres permettant la réalisation d'une semelle de gabarit moyen convenant à tous les pieds. De plus, on a observé que, de manière surprenante, les évidements situés sur la face inférieure de semelle ainsi que les éléments amovibles agencés dans au moins l'un des évidements peuvent présenter une hauteur ou une profondeur n'excédant pas quelques millimètres tout en procurant une bonne correction d'une abduction, d'une adduction, d'un varus, et/ou d'un valgus.

De même, les éléments de correction amovibles peuvent être maintenus dans les évidements situés sur la face inférieure de la semelle par tout moyen approprié tel que, par exemple, des moyens adhésifs ou du "velcro" (marque déposée) sans sortir du cadre de l'invention.

Enfin, il est évident que la semelle orthopédique conforme à l'invention peut être réalisée dans toute matière rigide courante, légèrement résiliente, soit par moulage, soit par modelage. Il va de soi que les exemples que l'on vient de donner ne sont que des illustrations particulières en aucun cas limitatives notamment quant aux domaines d'application de l'invention.

## Revendications

1. Semelle orthopédique proprioceptive modulaire pour le rétablissement et/ou la préservation d'une dynamique correcte de la marche ou de la course, ladite semelle étant obtenue dans un matériau élastique, **caractérisée en ce qu'**elle comporte d'une part, sur sa face supérieure, des moyens (1) pour solliciter, dès l'attaque du pas habituellement effectué par le talon, les récepteurs articulaires situés entre l'astragale et le calcanéum et des moyens (3,4,5) guidant le déroulement du pied sur l'axe physiologique de la marche et d'autre part, sur sa face inférieure, au moins un élément de correction amovible (11,12,13,14) apte à procurer une abduction ou une adduction lors d'une ouverture de pas déficitaire ou respectivement trop importante et/ou à corriger un varus, et/ou un valgus.

2. Semelle orthopédique suivant la revendication précédente **caractérisée en ce que** la semelle comprend sur sa face inférieure au moins un évidement (7,8,9,10) situé le long du bord interne et/ou externe de ladite semelle et dans lequel l'élément de correction amovible (11 à 14) obtenu dans un matériau plus rigide que le matériau de la semelle et de forme correspondant à la forme de l'évidement (7 à 10), est susceptible d'être positionné.

3. Semelle orthopédique suivant la revendication 2 **caractérisé en ce que** la section de l'évidement (7 à 10) croît depuis le bord interne ou externe de la semelle en direction de la partie médiane de cette dernière sur une courte distance, puis décroît.

4. Semelle orthopédique suivant l'une quelconque des revendications 2 ou 3 **caractérisé en ce que** la paroi de l'évidement (7 à 10) est inclinée vers l'intérieur dudit évidement depuis le fond jusqu'au bord de ce dernier afin de former une lèvre (15) en bordure de l'évidement (7 à 10) et **en ce que** l'élément amovible (11 à 14) comprend à sa périphérie un chanfrein (16) de sorte que la lèvre (15) maintienne en position l'élément amovible dans l'évidement (7 à 10), ledit élément étant introduit dans l'évidement par déformation élastique de la lèvre (15).

5. Semelle orthopédique suivant l'une quelconque des revendications 2 ou 3 **caractérisé en ce que** l'élément amovible (11 à 14) comprend sur l'une de ses faces un organe mâle (17) de fixation apte à coopérer avec un organe femelle (18) positionné au fond de l'évidement (7 à 10) afin de procurer l'attachement de l'élément amovible dans l'évidement.

6. Semelle orthopédique suivant l'une quelconque des revendications 2 à 5 **caractérisé en ce qu'**elle comprend un évidement anti-abduction (7) situé le long du bord interne de la semelle et s'étendant depuis l'arc de la voûte plantaire jusqu'au gros orteil du pied.

7. Semelle orthopédique suivant l'une quelconque des revendications 1 à 5 **caractérisé en ce qu'**elle comprend un évidement anti-adduction (8) situé le long du bord externe de la semelle et s'étendant depuis la voûte plantaire jusqu'à la zone pulpaire du petit doigt de pied.

8. Semelle orthopédique suivant l'une quelconque des revendications 2 à 7 **caractérisé en ce qu'**elle comprend un évidement anti-valgus (10) ou anti-pronation situé le long du bord interne de la semelle sous la voûte plantaire.

9. Semelle orthopédique suivant l'une quelconque des revendications 2 à 8 **caractérisé en ce qu'**elle comprend un évidement anti-varus (9) ou anti-supination situé le long du bord externe de la semelle et s'étendant depuis le cuboïde jusqu'au second évidement anti-adduction (8).

10. Semelle orthopédique suivant l'une quelconque des revendications précédentes **caractérisées en ce qu'**elle est pourvue, sur sa face supérieure, d'un canal profilé longitudinal sous-calcanéen, ou console (1), et d'épaisseur croissant depuis le talon jusqu'à une zone située sensiblement à l'aplomb vertical du col de l'astragale.

11. Semelle orthopédique suivant la revendication 10 **caractérisé en ce qu'**elle comporte, sur sa face supérieure, un élément profilé sous-scaphoïdien (3) qui présente sensiblement la forme d'une hémicoupole prolongeant la console (1) vers l'intérieur du pied.

12. Semelle orthopédique suivant l'une quelconque des revendications 10 ou 11 **caractérisé en ce qu'**elle comporte, sur sa face supérieure, un élément sous-cuboïdien (4) situé du côté externe de l'élément sous-scaphoïdien (3) et présentant la forme d'une graine de haricot, avec sa convexité tournée vers l'arrière à environ 45° de l'axe longitudinal médian de la semelle.

13. Semelle orthopédique suivant la revendication 11 **caractérisé en ce qu'**elle comporte, sur sa face supérieure, un moyen axial médiotarsien (5) de forme obovale et s'élargissant vers l'avant pour se terminer juste devant les têtes métatarsiennes afin de répartir l'appui sous les palettes métatarsiennes.

14. Semelle orthopédique suivant l'une quelconque des revendications précédentes **caractérisées en ce qu'**elle comporte, sur sa face inférieure, un sillon (19) en forme de serpentin qui s'étend sensiblement depuis le plat du pied jusqu'au talon et qui comprend des trous (20) régulièrement espacés débouchant sur la face supérieure de la semelle afin de permettre l'évacuation de la transpiration.

15. Semelle orthopédique suivant l'une quelconque des revendications 1 à 14 **caractérisé en ce que** le talon et l'avant pied de la semelle présente une structure alvéolaire.

16. Semelle orthopédique suivant la revendication 15 **caractérisé en ce que** la structure alvéolaire consiste dans une structure en nid d'abeille.

17. Semelle orthopédique suivant l'une quelconque des revendications 15- ou 16 **caractérisé en ce qu'**elle présente dans une zone talonnière postero-externe et/ou dans une zone des têtes métatarsiennes (23) et/ou dans une zone pulpaire sous le gros orteil (24) des alvéoles de plus petite taille que les alvéoles du reste de la semelle.

18. Semelle orthopédique suivant l'une quelconque des revendications précédentes **caractérisées en ce que** les éléments profilés (1,3,4,5) positionnés sur la face supérieure de la semelle et les évidements (7 à 10) positionnés sur la face inférieure de ladite semelle ainsi que les éléments amovibles (11 à 14) agencés dans au moins l'un des évidements présentent une hauteur ou une profondeur n'excédant pas quelques millimètres.

## Patentansprüche

1. Propriozeptive orthopädische modulare Einlage zur Wiederherstellung und/oder Aufrechterhaltung einer korrekten Gang- oder Laufdynamik, wobei diese Einlage aus einem elastischen Material angefertigt wird, **dadurch gekennzeichnet, dass** sie einerseits auf ihrer Oberseite Mittel (1) zur Inanspruchnahme von Gelenkrezeptoren umfasst, die zwischen dem Sprungbein und dem Fersenbein liegen, sobald ein Schritt ausgeführt wird, was üblicherweise mit der Ferse beginnt, und Mittel (3, 4, 5), die das Abrollen des Fußes auf der physiologischen Gangachse lenken, und andererseits auf ihrer Unterseite mindestens ein bewegliches Korrekturelement (11, 12, 13, 14), das in der Lage ist, bei einer zu kleinen oder zu großen Schrittöffnung ein Abspreizen oder ein Heranziehen hervorzurufen und/oder O- und/oder X-Beinigkeit zu korrigieren.

2. Orthopädische Einlage nach vorhergehendem Anspruch, **dadurch gekennzeichnet, dass** die Einlage auf ihrer Unterseite am inneren und/oder äußeren Rand dieser Einlage mindestens eine Aussparung (7, 8, 9, 10) aufweist, in die das lösbare Korrekturelement (11 bis 14), das aus einem Werkstoff besteht, der fester ist als der Werkstoff der Einlage und das in seiner Form der Form der Aussparung (7 bis 10) entspricht, positioniert werden kann.

3. Orthopädische Einlage nach Anspruch 2, **dadurch gekennzeichnet, dass** der Querschnitt der Aussparung (7 bis 10) vom inneren oder äußeren Rand der Einlage aus in Richtung ihres Mittelteils über eine kurze Strecke zunimmt und dann abnimmt.

4. Orthopädische Einlage nach einem der vorhergehenden Ansprüche 2 oder 3, **dadurch gekennzeichnet, dass** die Seitenwand der Aussparung (7 bis 10) vom Boden bis zu ihrer Kante in Innenrichtung dieser Aussparung geneigt ist, um am Rand der Aussparung (7 bis 10) eine Lippe (15) zu bilden, und dadurch, dass das bewegliche Element (11 bis 14) an seiner Umfangslinie eine Abschrägung (16) aufweist, so dass die Lippe (15) das bewegliche Element in der Aussparung (7 bis 10) in Position hält, wobei dieses Element durch elastische Verformung der Lippe (15) in die Aussparung eingeführt wird.

5. Orthopädische Einlage nach einem der Ansprüche 2 oder 3, **dadurch gekennzeichnet, dass** das bewegliche Element (11 bis 14) auf einer seiner Seiten ein Steckteil (17) zur Befestigung aufweist, das mit einem am Boden der Aussparung (7 bis 10) positionieren Aufnahmeteil (18) zusammenwirkt, um die Befestigung des beweglichen Elements in der Aussparung zu bewerkstelligen.

6. Orthopädische Einlage nach einem der Ansprüche 2 bis 5, **dadurch gekennzeichnet, dass** sie eine dem Abspreizen entgegenwirkende Aussparung (7) aufweist, die sich am Innenrand der Einlage befindet und sich vom Bogen des Fußgewölbes bis zum großen Zeh des Fußes erstreckt.

7. Orthopädische Einlage nach einem der Ansprüche 1 bis 5, **dadurch gekennzeichnet, dass** sie eine dem Heranziehen entgegenwirkende Aussparung (8) aufweist, die sich am Außenrand der Einlage befindet und sich vom Fußgewölbe bis zum pulpösen Bereich des kleinen Zehs des Fußes erstreckt.

8. Orthopädische Einlage nach einem der Ansprüche 2 bis 7, **dadurch gekennzeichnet, dass** sie eine O-Beinigkeit oder einer Einwärtsdrehung entgegenwirkende Aussparung (10) aufweist, die sich am Innenrand der Einlage unter dem Fußgewölbe befindet.

9. Orthopädische Einlage nach einem der Ansprüche 2 bis B, **dadurch gekennzeichnet, dass** sie eine X-Beinigkeit oder einer Auswärtsdrehung entgegenwirkende Aussparung (9) aufweist, die sich am Außenrand der Einlage befindet und sich vom Würfelbein bis zur zweiten, einem Heranziehen entgegenwirkenden Aussparung (8) erstreckt.

10. Orthopädische Einlage nach einem der vorhergehenden Ansprüche, **dadurch gekennzeichnet, dass** sie auf ihrer Oberseite unter dem Fersenbein mit einem Längs-Profilkanal oder einer Konsole (1) mit von der Ferse bis zu einem Bereich etwa senkrecht zum Sprungbeinhals zunehmender Dicke versehen ist.

11. Orthopädische Einlage nach Anspruch 10, **dadurch gekennzeichnet, dass** sie auf ihrer Oberseite unter dem Kahnbein ein Profilelement (3) aufweist, das etwa wie eine Halbkuppel geformt ist und die Konsole (1) in das Innere des Fußes verlängert.

12. Orthopädische Einlage nach einem der vorhergehenden Ansprüche 10 oder 11, **dadurch gekennzeichnet, dass** sie auf ihrer Oberseite unter dem Würfelbein ein Element (4) aufweist, das sich an der Außenseite des unter dem Kahnbein liegenden Elements (3) befindet und die Form eines Bohnensamens hat, wobei seine konvexe Wölbung in etwa 45 Grad zur Mittellängsachse der Einlage nach hinten gerichtet ist.

13. Orthopädische Einlage nach Anspruch 11, **dadurch gekennzeichnet, dass** sie auf ihrer Oberseite ein axiales mediotarsiales obovales Mittel (5) aufweist, das sich nach vorn verbreitert und genau vor den Mittelfußköpfen endet, um die Abstützung unter den Mittelfußplatten zu verteilen.

14. Orthopädische Einlage nach einem der vorhergehenden Ansprüche, **dadurch gekennzeichnet, dass** sie auf ihrer Unterseite eine wie eine Serpentine geformte Rille (19) aufweist, die sich etwa von der Fußfläche bis zur Ferse erstreckt und die regelmäßig beabstandete Löcher (20) aufweist, die auf der Oberseite der Einlage münden, um Schweiß abzuleiten.

15. Orthopädische Einlage nach einem der Ansprüche 1 bis 14, **dadurch gekennzeichnet, dass** die Ferse und der Vorderfuß der Einlage eine Zellstruktur haben.

16. Orthopädische Einlage nach Anspruch 15, **dadurch gekennzeichnet, dass** die Zellstruktur in einer Bienenwabenstruktur besteht.

17. Orthopädische Einlage nach einem der Ansprüche 15 oder 16, **dadurch gekennzeichnet, dass** sie einem posteroexternen Fersenbereich und/oder einem Bereich der Mittelfußköpfe (23) und/oder in einem pulpösen Bereich unter dem großen Zeh (24) Zellen hat, die kleiner sind als die Zellen auf der übrigen Einlage.

18. Orthopädische Einlage nach einem der vorhergehenden Ansprüche, **dadurch gekennzeichnet, dass** die Profilelemente (1, 3, 4, 5) auf der Oberseite der Einlage und die Aussparungen (7 bis 10) auf der Unterseite dieser Einlage sowie die beweglichen Elemente (11 bis 14), die mindestens in einer der Aussparung angeordnet sind, eine Höhe oder eine Tiefe haben, die einige Millimeter nicht überschreitet.

## Claims

1. Modular proprioceptive orthopaedic sole for re-establishing and/or preserving proper dynamics of walking or running, said sole being obtained in an elastic material, **characterized in that** it includes on its upper face, means (1) on the one hand for exerting force from the start of the stepping usually performed by the heel, on articular receptors located between the astragalus and the calcaneum and means (3, 4, 5) guiding the progress of the foot on the physiological axis of walking and on the other hand, on its lower face, at least one removable correcting component (11, 12, 13, 14) able to provide abduction or adduction upon a deficient or respectively too large step opening and/or to correct a varus, and/or a valgus.

2. The orthopaedic sole according to the preceding claim, **characterized in that** the sole comprises on its lower face at least one recess (7, 8, 9, 10) located along the internal and/or external edge of said sole, and in which the removable correcting component (11-14) obtained in a stiffer material than the material of the sole and with a shape corresponding to the shape of the recess (7-10) is capable of being positioned.

3. The orthopaedic sole according to claim 2, **characterized in that** the section of the recess (7-10) increases from the internal or external edge of the sole towards the median portion of the latter over a short distance, and then decreases.

4. The orthopaedic sole according to any of claims 2 or 3, **characterized in that** the wall of the recess (7-10) is tilted towards the inside of said recess from the bottom up to the edge of the latter in order to from a lip (15) bordering the recess (7-10) and **in that** the removable component (11-14) comprises at its periphery, a chamfer (16) so that the lip (15) maintains the removable component in position in the recess (7-10), said component being introduced into the recess by elastic deformation of the lip (15).

5. The orthopaedic sole according to any of claims 2 or 3, **characterized in that** the removable component (11-14) comprises on one of its faces, a male attachment unit (17) capable of cooperating with a female unit (18) positioned at the bottom of the recess (7-10) in order to provide attachment of the removable component in the recess.

6. The orthopaedic sole according to any of claims 2 or 5, **characterized in that** it comprises an anti-abduction recess (7) located along the internal edge of the sole and extending from the arc of the arch of the foot up to the big toe of the foot.

7. The orthopaedic sole according to any of claims 1 to 5, **characterized in that** it comprises an anti-abduction recess (8) located along the external edge of the sole and extending from the arch of the foot to the pulpal area of the little toe of the foot.

8. The orthopaedic sole according to any of claims 2 to 7, **characterized in that** it comprises an anti-valgus (10) or anti-pronation recess located along the internal edge of the sole under the arch of the foot.

9. The orthopaedic sole according to any of claims 2 to 8, **characterized in that** it comprises an anti-varus (9) or anti-supination recess located along the external edge of the sole, and extending from the cuboid bone up to the second anti-abduction recess (8).

10. The orthopaedic sole according to any of the preceding claims, **characterized in that** it is provided, on its upper face, with a sub-calcaneal longitudinal profiled channel, or console (1), and with increasing thickness from the heel up to an area substantially located at the vertical of the neck of the astragalus.

11. The orthopaedic sole according to claim 10, **characterized in that** it includes, on its upper face, a sub-scaphoidal profiled component (3) which substantially has the shape of a half-dome extending the console (1) towards the inside of the foot.

12. The orthopaedic sole according to any of claims 10 or 11, **characterized in that** it includes, on its upper face, a sub-cuboidal component (4), located on the external side of the sub-scaphoidal component (3) and having the shape of a bean, with its convexity turned towards the rear at about 45° from the median longitudinal axis of the sole.

13. The orthopaedic sole according to claim 11, **characterized in that** it includes, on its upper face, a mediotarsal axial means (5) with an oboval shape and widening frontward so as to end just in front of the metatarsal heads so as to distribute the pressure under the metatarsal foot plates.

14. The orthopaedic sole according to any of the preceding claims, **characterized in that** it includes, on its lower face, a coil-shaped sulcus (19) which substantially extends from the foot plate to the heel and which comprises regularly spaced out holes (20) opening onto the upper face of the sole to allow sweat to be discharged.

15. The orthopaedic sole according to any of claims 1 to 14, **characterized in that** the heel and the forepart of the sole has a cellular structure.

16. The orthopaedic sole according to claim 15, **characterized in that** the cellular structure consists of a honeycomb structure.

17. The orthopaedic sole according to any of claims 15 or 16, **characterized in that** it has in a postero-external heel area and/or in an area of metatarsal heads (23) and/or in a pulpal area under the big toe (24), cells of a smaller size than the cells of the remainder of the sole.

18. The orthopaedic sole according to any of the preceding claims, **characterized in that** the profile components (1, 2, 4, 5) positioned on the upper face of the sole and the recesses (7-10) positioned on the lower face of said sole as well as the removable components (11-14) arranged in at least one of the recesses has a height or a depth not exceeding a few millimetres.
